# EUROPEAN PATENT APPLICATION

(11) **EP 4 253 362 A1**
(43) Date of publication of application: **04.10.2023**
(21) Application number: 21898417.7
(22) Date of filing: 05.11.2021
(51) Int. Cl.: C07C 67/343, C07C 67/62, C07C 69/708

(54) **METHOD FOR PREPARING HETEROGENEOUS LINEAR CARBONATE BY USING ACID ION-EXCHANGE RESIN**

(30) Priority: 27.11.2020 KR 20200163325
(71) Applicant: Lotte Chemical Corporation, Seoul, 05551 (KR)
(72) Inventor: HAN, Eun Hye, Daejeon 34110 (KR); KIM, Wang Gyu, Daejeon 34110 (KR); BAEK, Mi Hwa, Daejeon 34110 (KR); CHOI, Jong Myung, Daejeon 34110 (KR); KIM, Jin Hyung, Daejeon 34110 (KR)
(74) Representative: Germain Maureau
(86) International application number: PCT/KR2021/016027
(87) International publication number: WO 2022/114591

(57) **Abstract**

The present invention provides a method of preparing a heterogeneous linear carbonate, the method including transesterifying an aliphatic alcohol and a symmetric linear carbonate in the presence of a catalyst, wherein the catalyst is an acidic ion exchange resin, and a mass ratio of the aliphatic alcohol and the symmetric linear carbonate (aliphatic alcohol:symmetric linear carbonate) is 1:10 or more and 50:1 or less. The preparation method of the present specification provides a method of preparing a heterogeneous, symmetric linear carbonate and asymmetric linear carbonate in high yield without a process of reactive distillation, and is economical because a product and a catalyst can be easily separated.

## Description

### [Technical field]

The present specification relates to a preparation process that advantageously simultaneously obtains high yields of a heterogeneous linear carbonate, i.e. a symmetric linear carbonate and an asymmetric linear carbonate.

### [Background Art]

Linear carbonates such as ethyl methyl carbonate (EMC) and diethyl carbonate (DEC) are mainly used as solvents (electrolytes) for lithium secondary batteries, and the carbonates are mainly used as a solvent for lithium secondary batteries because the carbonates are superior in energy storage density, charge capacity, number of charge/discharge cycles, stability, and the like compared to conventional solvents.

As a method of preparing ethyl methyl carbonate and diethyl carbonate, a transesterification reaction of a mixture of cyclic carbonate (ethylene carbonate or propylene carbonate) and ethanol and methanol is generally well known. For example, when any dialkyl carbonate of diethyl carbonate, ethyl methyl carbonate and dimethyl carbonate is prepared by transesterifying ethylene carbonate or propylene carbonate and ethanol or a mixture of ethanol and methanol, methyl glycol ether or ethyl glycol ether is generated as a by-product.

These reaction by-products tend to be in an azeotropic state with a desired product and are very difficult to separate by distillation, so that it is common to purify the desired product by introducing a new extraction separation method.

Meanwhile, an industrial preparation method of ethyl methyl carbonate and diethyl carbonate by a transesterification reaction using dimethyl carbonate (DMC) as a raw material for preparation is also known. As an example, a reactive distillation method of dimethyl carbonate and ethanol using a basic catalyst such as sodium methoxide (SME) as a transesterification catalyst has a problem in that equipment and energy costs are high. In addition, sodium methoxide, which is a water-reactive material and is highly dangerous and hazardous, and does not dissolve in products, causing problems such as column plugging and fouling in equipment.

As another example, as the reactive distillation is a method of utilizing extractive distillation using a gel type strong basic exchange resin catalyst as a transesterification catalyst, since extractive distillation requires the addition of a separation process for separating an extractant into a product, there is a problem in that equipment costs and energy costs are high. In addition, deactivation proceeds rapidly due to the loss of exchange groups and formation of fine powders caused by low catalyst strength, and the internal pressure also rises, requiring a process of periodically introducing new catalysts, which reduces productivity and increases production costs.

Most of the methods of preparing ethyl methyl carbonate and diethyl carbonate using a cyclic carbonate or dimethyl carbonate (DMC) as raw materials are transesterification reactions using chemical equilibrium reactions. Then, in order to advance this reaction efficiently, a method of distilling off alkylene glycol and methanol which are reaction by-products is performed. However, at atmospheric pressure, for example, since it is practically impossible for DMC to form an azeotropic mixture with methanol, which has an approximate composition of 70% methanol and 30% DMC, and it is practically impossible to completely react DMC when performing this kind of transesterification reaction, in the actual reaction, DMC was also distilled off along with methanol, and as a result, the DMC conversion rate was insufficient or industrial reproducibility was low. Therefore, a separate method for separating and removing methanol from the azeotropic mixture of DMC and methanol is required.

Therefore, a new preparation method with a high DMC conversion rate is required.

### [Related Art Document]

### [Patent Document]

(Patent Document 1) Japanese Unexamined Patent Publication No. 2010-168365

### [Disclosure]

### [Technical Problem]

The present invention relates to a preparation method for advantageously obtaining heterogeneous linear carbonates, i.e., a symmetric linear carbonate and an asymmetric linear carbonate simultaneously, with high selectivity, by performing a transesterification reaction between an aliphatic alcohol and a symmetric linear carbonate at a high conversion rate without separate reactive distillation and distilling the symmetric linear carbonate out of the reaction system as an azeotropic mixture of reaction by-products.

### [Technical Solution]

The present invention provides a method of preparing a heterogeneous linear carbonate, the method including transesterifying an aliphatic alcohol and a symmetric linear carbonate in the presence of a catalyst, wherein the catalyst is an acidic ion exchange resin, and a mass ratio of the aliphatic alcohol and the symmetric linear carbonate (aliphatic alcohol:symmetric linear carbonate) is 1:10 or more and 50:1 or less.

### [Advantageous Effects]

A preparation method according to one embodiment of the present invention can simultaneously prepare a heterogeneous, symmetric linear carbonate and asymmetric linear carbonate in high selectivity and high yield without a process of reactive distillation.

In addition, the preparation method according to one embodiment of the present invention uses an acidic ion exchange resin as a catalyst to easily separate a product and a catalyst, thereby preparing a high-purity linear carbonate, and a separated catalyst is reusable and thus has high economic efficiency.

Lastly, the preparation method according to one embodiment of the present invention uses an acidic ion exchange resin, and unlike conventionally used catalysts, it is not water-reactive, so the process risk is low.

### [Modes of the Invention]

Advantages and features of the present invention and methods of achieving them will become apparent with reference to the embodiments described below specifically. However, the present invention is not limited to the embodiments disclosed below and may be embodied in various different forms, and the embodiments merely serve to complete the disclosure of the present invention and to fully inform the scope of the invention to those skilled in the art to which the invention pertains, which is defined only by the spirit of the claims. Throughout the specification, like reference numerals refer to like components.

Unless otherwise defined, all terms (including technical and scientific terms) used in the present specification may be used in a meaning commonly understood by those of ordinary skill in the art to which the present invention belongs. In addition, terms defined in commonly used dictionaries are not interpreted ideally or excessively unless explicitly specifically defined.

Throughout the specification, when a part is said to "include" a component, this means that the part may further include other components rather than excluding other components unless specifically stated to the contrary.

Hereinafter, the present invention will be described in detail.

The present invention provides a method of preparing a heterogeneous linear carbonate, the method including transesterifying an aliphatic alcohol and a symmetric linear carbonate in the presence of a catalyst, wherein the catalyst is an acidic ion exchange resin, and a mass ratio of the aliphatic alcohol and the symmetric linear carbonate (aliphatic alcohol:symmetric linear carbonate) is 1:10 or more and 50:1 or less.

In the present specification, the aliphatic alcohol is not particularly limited, but may include alcohols having 1 to 10 carbon atoms, specifically methanol, ethanol, propanol, isopropanol, butanol, isobutanol, and the like. In one embodiment of the present specification, the aliphatic alcohol is ethanol.

In one embodiment of the present invention, the symmetric linear carbonate, which is a reactant in the transesterification reaction, is dimethyl carbonate (DMC).

In one embodiment of the present invention, heterogeneous linear carbonates produced after the transesterification reaction are symmetric linear carbonates and asymmetric linear carbonates.

In one embodiment, the asymmetric linear carbonate produced after the transesterification reaction is ethyl methyl carbonate (EMC).

In one embodiment, the symmetric linear carbonate produced after the transesterification reaction is diethyl carbonate (DEC).

According to one embodiment of the present invention, the transesterification reaction produces a heterogeneous linear carbonate, namely the asymmetric linear carbonate and the asymmetric linear carbonate. The asymmetric linear carbonate of the product in the transesterification reaction is ethyl methyl carbonate (EMC), and the symmetric linear carbonate is diethyl carbonate (DEC).

In one embodiment of the present invention, the acidic ion exchange resin is a strongly acidic ion exchange resin. In the present specification, strongly acidic means a strong electrolyte that is completely ionized in an aqueous solution, and is mostly present in an ionic state, and examples may include sulfuric acid (H₂SO₄), nitric acid (HNO₃), hydrochloric acid (HCl), hydrobromic acid (HBr), hydroiodic acid (HI), and perchloric acid (HClO₄). Weakly acidic may mean a weak electrolyte that is only partially ionized in an aqueous solution.

In one embodiment of the present invention, the acidic ion exchange resin may be a bead type. The bead type may be a gel type or a porous type.

In the preparation method according to one embodiment of the present invention, high conversion of the symmetric linear carbonate and high selectivity of the asymmetric linear carbonate can be secured by using the acidic ion exchange resin.

In addition, the preparation method according to one embodiment of the present invention uses an acidic ion exchange resin to easily separate a product and a catalyst, thereby preparing a high-purity heterogeneous symmetric linear carbonate and asymmetric linear carbonate, and a separated catalyst is reusable and thus has high economic efficiency.

Lastly, the preparation method according to one embodiment of the present invention uses an acidic ion exchange resin, and unlike conventionally used catalysts, it is not water-reactive, so the process risk is low.

In one embodiment of the present specification, in the transesterification reaction, a mass ratio of the aliphatic alcohol and the symmetric linear carbonate (aliphatic alcohol:symmetric linear carbonate) is 1:10 or more and 50:1 or less. In one embodiment, in the transesterification reaction of the aliphatic alcohol and the symmetric linear carbonate, a content of the symmetric linear carbonate may be 0.4 parts by mass or more and 4 parts by mass or less, based on 1 part by mass of the aliphatic symmetric linear carbonate. When a mass ratio of the aliphatic alcohol and the symmetric linear carbonate is less than 1:10, since the amount of aliphatic alcohol is small, the symmetric linear carbonate remains in excess, and the catalyst is not sufficiently swelled, the contact with the catalyst is lowered and the efficiency is lowered, resulting in a decrease in productivity, and when the mass ratio of the aliphatic alcohol and the symmetric linear carbonate exceeds 50: 1, after the reaction, a size of the distillation process to separate the asymmetric linear carbonate (EMC) and aliphatic alcohol increases, and as the aliphatic alcohol and asymmetric linear carbonate (EMC) are azeotropes, the larger the amount of aliphatic alcohol, the more columns with high separation efficiency are required, a lot of energy is consumed in the decompression and/or pressurization process, a large amount of asymmetric linear carbonate (EMC) is lost to the top of the distillation process, which makes the process unproductive.

In one embodiment of the present invention, the catalyst is an acidic ion exchange resin having an exchange capacity of 1 (eq/l-wet resin) or more and 2.5 (eq/l-wet resin) or less. In general, the higher the degree of crosslinking of the ion exchange resin, the more exchange groups are attached to the matrix, so that the exchange capacity tends to be high, but the reaction efficiency decreases because the exchange groups participating in the reaction decrease due to a strong bond between the matrix and the exchange groups. When the exchange capacity of the catalyst exceeds 2.5 (eq/l-wet resin), the reaction rate is too slow, and when the exchange capacity of the catalyst is less than 1 (eq/l-wet resin), the reaction rate is fast, but the bond between the matrix and the exchange groups is weakened, resulting in a decrease in strength against rapid volume change.

In the present specification, the exchange capacity means that the ability of an ion exchange resin to adsorb ions is expressed as an equivalent amount of ions obtained by adsorbing a certain volume of the resin, that is, may mean an exchange amount that can be removed by 1 liter of resin.

In the present specification, the unit of the exchange capacity is eq/l-wet resin, and means a value expressed as an equivalent of exchange capacity per liter of the ion exchange resin.

In one embodiment of the present specification, an exchange group of the acidic ion exchange resin includes a sulfonate group or a carboxylate group. Specifically, the acidic ion exchange resin is obtained by binding a sulfonic acid group (-SO₃H) and a carboxyl group (-COOH) as an exchange group to a basic polymer matrix having a network structure, and exchanges cations such as Ca²⁺, Na⁺, and H⁺.

In one embodiment of the present specification, the matrix of the acidic ion exchange resin is a copolymer of styrene and divinylbenzene. In one embodiment of the present invention, the matrix of the acidic ion exchange resin may have a low crosslinking degree or a high crosslinking degree. In the present specification, the crosslinking degree of the ion exchange resin is also referred to as % of divinylbenzene, and can usually be divided into a low crosslinking degree and a high crosslinking degree based on a crosslinking degree of 8%.

In one embodiment, the transesterification reaction is performed at a temperature of 30 °C or more and 110 °C or less. More specifically, the transesterification reaction is performed at a temperature of 70 °C or more and 100 °C or less. When the transesterification reaction is performed below 30 °C, there is a problem that the activity of the catalyst is lowered and a reaction rate is lowered, and when the transesterification reaction is performed above 110 °C, the exchange group of the acidic ion exchange resin is decomposed and deteriorated, so that the exchange group becomes weakly acidic, which can lead to a decrease in exchange capacity.

In one embodiment of the present invention, the catalyst is input at 10% by weight or more and 1,000% by weight or less based on the weight of the symmetric linear carbonate. In one embodiment, in the transesterification reaction, the catalyst is input at 10% by weight or more and 500% by weight or less based on the weight of the symmetric linear carbonate. In the case of the catalyst being input in an amount exceeding 1,000% by weight based on the weight of the symmetric linear carbonate, when an active group in contact with the reactants increases, showing high selectivity and conversion, but an excessive amount of catalyst is input into a reactor, there is a problem in that operating costs and production costs increase due to an increase in an internal pressure of the reactor and an increase in washing time of the ion exchange resin.

In one embodiment of the present invention, the transesterification reaction is performed in a fixed bed reactor or a continuous stirred tank reactor (CSTR).

In one embodiment of the present invention, the transesterification reaction is performed in the fixed bed reactor. In this case, operation is simple and energy costs can be saved. In addition, separation of a product is easy by reducing the generation of precipitates generated in a separation process.

Meanwhile, the method of preparing a heterogeneous linear carbonate according to the present invention may further include conventional steps known in the art before or after each of the above steps, in addition to the above steps.

Hereinafter, examples will be given to describe the present invention in detail. However, the examples according to the present invention may be modified in various other forms, and the scope of the present invention is not to be construed as being limited to the examples described below. The examples of the present specification are provided to more completely explain the present invention to those of ordinary skill in the art.

### Example 1

14.74 g of ethanol and 36.03 g of dimethyl carbonate were reacted in the presence of 144.12 g of CMP08LH (catalyst; exchange capacity: > 1.1 (eq/l); matrix: Styrene-DVB (low crosslinking degree, 4%); exchange group: sulfonic acid group) at 75 °C for 3 hours at a stirring speed of 200 rpm in a CSTR reactor.

### Example 2

The reaction was performed in the same manner as in Example 1, except that 27.64 g of ethanol was used instead of 14.74 g of ethanol in Example 1.

### Example 3

The reaction was performed in the same manner as in Example 1, except that 36.03 g of ethanol was used instead of 14.74 g of ethanol in Example 1.

### Example 4

The reaction was performed in the same manner as in Example 1, except that 73.71 g of ethanol was used instead of 14.74 g of ethanol in Example 1.

### Example 5

The reaction was performed in the same manner as in Example 1, except that 147.42 g of ethanol was used instead of 14.74 g of ethanol in Example 1.

### Comparative Example 1

The reaction was performed in the same manner as in Example 1, except that in Example 1, 0.108 g of sodium methoxide (SME) was used instead of 144.12 g of CMP08LH, 27.64 g of ethanol was used instead of 14.74 g of ethanol, and the reaction was performed at 70 °C for 1 hour instead of 75 °C for 3 hours.

### Comparative Example 2

The reaction was performed in the same manner as in Comparative Example 1, except that 36.03 g of ethanol was used instead of 27.64 g of ethanol in Comparative Example 1.

### Comparative Example 3

The reaction was performed in the same manner as in Comparative Example 1, except that 73.71 g of ethanol was used instead of 27.64 g of ethanol in Comparative Example 1.

### Comparative Example 4

The reaction was performed in the same manner as in Comparative Example 1, except that 147.42 g of ethanol was used instead of 27.64 g of ethanol in Comparative

### Example 1.

### Comparative Example 5

The reaction was performed in the same manner as in Comparative Example 4, except that in Comparative Example 4, 0.180 g of sodium hydroxide (NaOH) was used instead of sodium methoxide, and the reaction was performed at 75 °C instead of 70 °C.

### Comparative Example 6

The reaction was performed in the same manner as in Comparative Example 4, except that in Comparative Example 4, 0.180 g of potassium hydroxide (KOH) was used instead of sodium methoxide, and the reaction was performed at 75 °C instead of 70 °C.

The reaction results of Examples 1 to 5 and Comparative Examples 1 to 6 are shown in Table 1 below.

**[Table 1]**

| Classification | EtOH/DMC (mass ratio) | Reaction results (%) | | |
|---|---|---|---|---|
| | | DMC Conversion Rate | EMC selectivity | DEC selectivity |
| Example 1 | 0.4 | 63 | 77 | 23 |
| Example 2 | 0.8 | 72 | 75 | 25 |
| Example 3 | 1 | 80 | 67 | 33 |
| Example 4 | 2 | 87 | 59 | 41 |
| Example 5 | 4 | 95 | 41 | 59 |
| Comparative Example 1 | 0.8 | 65 | 76 | 24 |
| Comparative Example 2 | 1 | 72 | 70 | 30 |
| Comparative Example 3 | 2 | 87 | 56 | 44 |
| Comparative Example 4 | 4 | 90 | 61 | 39 |
| Comparative Example 5 | 4 | 95 | 38 | 62 |
| Comparative Example 6 | 4 | 95 | 38 | 62 |

In Table 1, DMC means dimethyl carbonate, EMC means ethyl methyl carbonate, and DEC means diethyl carbonate. Referring to Table 1, it was confirmed that in the case of Example 5 in which the mass ratio of an aliphatic alcohol (ethanol) and a symmetric linear carbonate (dimethyl carbonate) is 4, the conversion rate of dimethyl carbonate tends to increase by 30% or more compared to Example 1 having a mass ratio of 0.4. Therefore, in the preparation method according to one embodiment of the present invention, it was confirmed that the higher the content of aliphatic alcohol, the higher the DMC conversion rate. However, when the content of ethanol is excessive, since the amount of catalyst compared to the total amount of raw materials may be reduced and reactivity may be lowered, it is necessary to appropriately adjust the content of aliphatic alcohol.

### Example 6

46.65 g of ethanol and 60.08 g of dimethyl carbonate were reacted in the presence of CMP08LH (catalyst) at 70 °C for 3 hours at a stirring speed of 200 rpm in a CSTR reactor.

### Example 7

The reaction was performed in the same manner as in Example 6, except that PCC40LH was used instead of CMP08LH in Example 6.

### Example 8

The reaction was performed in the same manner as in Example 6, except that SPC180H was used instead of CMP08LH in Example 6.

### Example 9

The reaction was performed in the same manner as in Example 6, except that SPC260H was used instead of CMP08LH in Example 6.

### Example 10

The reaction was performed in the same manner as in Example 6, except that SPC320H was used instead of CMP08LH in Example 6.

The reaction results of Examples 6 to 10 are shown in Table 2 below.

**[Table 2]**

| Classificati on | Catalyst name | Physical properties | | Reaction results (%) | | |
|---|---|---|---|---|---|---|
| | | Exchange capacity (eq/l) | Matrix (DVB, %) | DMC conversion rate | EMC selectivity | DEC selectivity |
| Example 6 | CMP08 LH | > 1.1 | Styrene-DVB (low crosslinking degree, 4%) | 50 | 98 | 2 |
| Example 7 | PCC40 LH | > 1.2 | Styrene-DVB (low crosslinking degree, 4%) | 48 | 96 | 4 |
| Example 8 | SPC180 | > 1.5 | Styrene-DVB | 44 | 100 | 0 |
| | H | | (high crosslinking degree, 10∼20%) | | | |
| Example 9 | SPC260 H | > 1.8 | | 42 | 100 | 0 |
| Example 10 | SPC320 H | > 1.9 | | 36 | 100 | 0 |

Referring to Table 2, it was confirmed that the transesterification reaction rate decreased as the exchange capacity of the ion exchange resin increased, and the exchange capacity was closely related to the activity of the transesterification catalyst.

### Example 11

14.74 g of ethanol and 36.03 g of dimethyl carbonate were reacted in the presence of 90.08 g of CMP08LH at 70 °C for 3 hours at a stirring speed of 200 rpm in a CSTR reactor.

### Example 12

The reaction was performed in the same manner as in Example 11, except that the reaction was performed at 80 °C instead of 70 °C in Example 11.

### Example 13

The reaction was performed in the same manner as in Example 11, except that the reaction was performed at 90 °C instead of 70 °C in Example 11.

### Comparative Example 7

The reaction was performed in the same manner as in Example 11, except that in Example 1, 0.0162 g of sodium methoxide (SME) was used instead of 90.08 g of CMP08LH, 27.64 g of ethanol was used instead of 14.74 g of ethanol, and the reaction was performed for 1 hour instead of 3 hours.

### Comparative Example 8

The reaction was performed in the same manner as in Comparative Example 7, except that the reaction was performed at 100 °C instead of 70 °C in Comparative Example 7.

### Comparative Example 9

The reaction was performed in the same manner as in Comparative Example 7, except that the reaction was performed at 130 °C instead of 70 °C in Comparative Example 7.

The results of Examples 11 to 13 and Comparative Examples 7 to 9 are shown in Table 3 below.

**[Table 3]**

| Classification | | Example 11 | Example 12 | Example 13 | Compara tive Example 7 | Compara tive Example 8 | Compara tive Example 9 |
|---|---|---|---|---|---|---|---|
| Reaction results | DMC conversion rate (%) | 54 | 64 | 69 | 49 | 57 | 65 |
| | EMC selectivity (%) | 93 | 85 | 77 | 90 | 86 | 79 |
| | DEC selectivity (%) | 7 | 15 | 23 | 10 | 14 | 21 |

Referring to the results of Table 3, it was confirmed that the higher the temperature, the higher the DMC conversion rate, and the higher the transesterification reaction rate.

With the results of Tables 1 to 3, it was possible to provide optimal conditions for a method of preparing an asymmetric linear carbonate having a high DMC conversion rate in the presence of the acidic ion exchange resin catalyst. In addition, compared to conventional catalysts, it can be reused without pre-treatment and is not water-reactive, so that it can provide a low process risk.

## Claims

1. A method of preparing a heterogeneous linear carbonate, comprising transesterifying an aliphatic alcohol and a symmetric linear carbonate in the presence of a catalyst,
wherein the catalyst is an acidic ion exchange resin, and
a mass ratio of the aliphatic alcohol to the symmetric linear carbonate (the aliphatic alcohol:the symmetric linear carbonate) is 1:10 or more and 50:1 or less.

2. The method of claim 1, wherein the catalyst is an acidic ion exchange resin having an exchange capacity of 1 (eq/l-wet resin) or more and 2.5 (eq/l-wet resin) or less.

3. The method of claim 1, wherein an exchange group of the acidic ion exchange resin includes a sulfonate group or a carboxylate group.

4. The method of claim 1, wherein a matrix of the acidic ion exchange resin is a copolymer of styrene and divinylbenzene.

5. The method of claim 1, wherein in the transesterification reaction, the catalyst is input at 10% by weight or more and 1,000% by weight or less based on the weight of the symmetric linear carbonate.

6. The method of claim 1, wherein the transesterification reaction is performed in a fixed bed reactor or a continuous stirred tank reactor (CSTR).
